# EUROPEAN PATENT APPLICATION

(11) **EP 4 129 264 A1**
(43) Date of publication of application: **08.02.2023**
(21) Application number: 21779770.3
(22) Date of filing: 31.03.2021
(51) Int. Cl.: A61H 3/00, A61F 5/01, A61B 5/11, A61B 5/103, A61H 1/02

(54) **ROBOTIC ORTHOSIS FOR LOWER EXTREMITY FOR GAIT REHABILITATION TRAINING**

(30) Priority: 31.03.2020 KR 20200039396
(71) Applicant: Seoul National University Hospital, Seoul 03080 (KR); Seoul National University R & DB Foundation, Seoul 08826 (KR)
(72) Inventor: PAIK, Nam-Jong, Seoul 05649 (KR); PARK, Jihong, Seongnam-si, Gyeonggi-do 13434 (KR); PARK, Yong-Lae, Seoul 06289 (KR); KWON, Junghan, Seoul 08816 (KR)
(74) Representative: V.O.
(86) International application number: PCT/KR2021/004012
(87) International publication number: WO 2021/201604

(57) **Abstract**

Disclosed is a robotic orthosis for a lower extremity for gait rehabilitation training, comprising a knee stretching member which is provided to be installable on the knee so as to enable the knee joint to be stretched in a swing phase and enable a state in which the knee is stretched to be maintained in a stance phase. The knee stretching member comprises: a knee sleeve surrounding the knee joint; and a knee supporting chamber which is mounted so as to be connected to the knee sleeve, and which, when air is introduced therein and is inflated in the swing phase, enables the knee to be stretched by supporting the knee joint, and enables the state in which the knee is stretched to be maintained in the stance phase.

## Description

### Technical Field

The present specification relates to a robotic orthosis for a lower extremity for gait rehabilitation training.
[National R&D Program for Supporting the Invention]
[Project Identification Number] 1711104112
[Project Number] 2016R1A5A1938472
[Ministry Name] Ministry of Science and ICT
[Project Management (Specialized) Institute Name] National Research Foundation of Korea
[Research Program Name] Fundamental Research Program in Science and Engineering Fields
[Research Project Name] Center for SoFT meta-Human
[Contribution Ratio] 1/1
[Project Performing Agency Name] Seoul National University R&DB Foundation
[Research Period] November 1, 2016 to December 31, 2022

### Background Art

Stroke is one of the main causes of death not only in Korea but also throughout the world, one of the main causes of adult disability, and a disease that causes a decline in gait, daily living activities, and cognition and lowers the quality of life, resulting in a large burden personally and socially to a patient A reduction in stroke mortality due to the medical advancement results in an increase in the number of patients suffering from sequelae of a cranial nerve disease.

For a lower extremity, hemiplegia causes a lack of physical (muscular) strength of an affected leg for supporting a patient's weight during walking, thus reducing gait stability, a lack of strength for generating thrust by kicking the ground, and a phenomenon of foot drop during a swing motion.

As a result, the patient suffers from gait abnormality which is characterized by hip hiking and circumduction gait as recompense motions. Asymmetry between both lower extremities not only disturbs gait rehabilitation training for a normal pattern but also is a principal cause of exhaustion of physical strength during walking and an increase in fall risk of a patient.

Currently, various types of orthoses are used in rehabilitation courses for hemiplegia patients. A fixed ankle orthosis for gait assistance can inhibit an ankle from excessive dropping, but does not sufficiently imitate motions of a joint during normal gait motions of a lower extremity, thus being a cause of degrading gait stability and symmetry.

In order to supplement such shortcomings described above, several items of equipment have been developed by adopting robot engineering for over a decade, and rehabilitation or the like using a robot having an exoskeleton structure is reported to have a partial clinical effect; however, most of the items of equipment are high in cost and large in volume, are often used only for repeating a simple motion, and have a problem of a limit in detecting a motion of a patient and providing a customized treatment protocol.

In addition, an existing orthosis or a rehabilitation apparatus does not include a sensing system suitable for accurately detecting a patient's gait condition, and thus an additional measurement instrument needs to be used in order to quantitatively analyze characteristics of the patient's gait and motion.

Consequently, it is not possible to provide feedback based on accurate evaluation of a condition, and thus it is difficult to perform quantitative rehabilitation training.

Such problems described above bring about a demand for a device that can assist a post-stroke patient's knee joint and ankle joint strength on one leg.

### [Prior Art Literature]

### [Patent Literature]

[Patent Literature 1] Korean Patent Registration No. 10-2018175

### Disclosure

### Technical Problem

According to an aspect of embodiments of the present invention, there is provided a mobile robotic orthosis for a lower extremity for gait rehabilitation training that is not stationary but can be applied during a walking process of the patient.

According to another aspect of the embodiments of the present invention, there is provided a robotic orthosis for a lower extremity for gait rehabilitation training that can assist a patient in a natural gait close to a normal gait by enabling a knee joint of a paraplegic patient such as a post-stroke hemiplegic patient to be extended, flexed, or the like according to a gait cycle of the patient.

According to still another aspect of the embodiments of the present invention, there is provided a robotic orthosis for a lower extremity for gait rehabilitation training that can be implemented as a soft wearable device even with a pump which supplies air pressure.

### Solution to Problem

According to exemplary embodiments of the present invention, there is provided a robotic orthosis for a lower extremity for gait rehabilitation training including: a knee stretching member which is provided to be installable on a knee so as to enable a knee joint to be stretched in a swing phase and enable a state in which the knee is stretched to be maintained in a stance phase. The knee stretching member comprises a knee sleeve surrounding the knee joint; and a knee supporting chamber which is mounted so as to be connected to the knee sleeve, and which, when air is introduced therein and is inflated in the swing phase, enables the knee to be stretched by supporting the knee joint, and enables the state in which the knee is stretched to be maintained in the stance phase.

According to an embodiment, the robotic orthosis for a lower extremity for gait rehabilitation training of the present invention may further include a luggage carrier that generates compressed air and supplies the generated compressed air to the knee stretching member.

According to the embodiment, desirably, the luggage carrier is an autonomous driving luggage carrier.

According to the embodiment, desirably, the knee sleeve and the knee supporting chamber are made of a soft material.

According to the embodiment, the robotic orthosis for a lower extremity for gait rehabilitation training of the present invention may further include at least one of a first ankle supporting member that is installed at an ankle so as to inhibit inversion or eversion bending of the ankle, and a second ankle supporting member that is installed on at least one of a shin and a calf such that an ankle is flexed toward a dorsum or a sole of a foot.

According to the embodiment, the first ankle supporting member may have: an ankle sleeve surrounding an ankle joint; and an ankle supporting chamber which is mounted so as to be connected to the ankle sleeve, and which, when air is introduced therein and is inflated, supports the ankle joint.

In addition, the second ankle supporting member may have: a shin sleeve surrounding a shin; guards that are installed at both the shin and the calf, respectively; and artificial muscle packs that are connected to the respective guards and have a plurality of air chambers inside.

The second ankle supporting member may further have: a Velcro tie that surrounds and secures the guards; and a buckle that is installed at a foot accommodating portion and combines the artificial muscle packs.

The air chambers may be installed at both sides of the shin and the calf. In a state in which a sole is in contact with ground, air in the air chamber installed at the side of the shin may be discharged outside, and air may be injected into the air chamber installed at the side of the calf. In a state in which a sole is separated from the ground, air may be injected into the air chamber installed at the side of the shin, and air in the air chamber installed at the side of the calf may be discharged outside.

According to the embodiment, the robotic orthosis for a lower extremity for gait rehabilitation training of the present invention may further include a luggage carrier having a compressor which generates compressed air and a line which supplies the generated compressed air to at least one of the knee stretching member and the first and second ankle supporting members.

According to the embodiment, the robotic orthosis for a lower extremity for gait rehabilitation training of the present invention may further include an inertial measurement module that is installed on at least one of a patient's thigh, shin, and dorsum of a foot and checks the patient's gait condition and gait pattern in real time based on an angle between ground and at least one of the thigh, the shin, and the dorsum of the foot during the patient's walking so as to control an operation of at least one of the knee stretching member and the first and second ankle supporting members.

Desirably, the inertial measurement module is capable of determining a gait phase as any one of a loading response phase, a mid stance phase, a terminal stance phase, a pre-swing phase, an initial swing phase, a mid swing phase, and a terminal swing phase based on a posture of the patient's both lower extremities and whether the patient's soles are in contact with the ground.

According to the embodiment, the luggage carrier may supply the compressed air to the knee stretching member in the terminal swing phase, the loading response phase, the mid stance phase, and the terminal stance phase and supply the compressed air to the first ankle supporting member in the terminal swing phase, the loading response phase, and the terminal stance phase.

### Advantageous Effects of Invention

A robotic orthosis for a lower extremity for gait rehabilitation training of the embodiments of the present invention enable a knee joint of a paraplegic patient such as a post-stroke hemiplegic patient to be extended, flexed, or the like according to a gait cycle of the patient when the patient walks. In particular, the robotic orthosis includes a pneumatic artificial muscle pack so as to be capable of providing active assisting strength when compressed air is injected, so it is possible to assist and support behavior of a knee joint and behavior of an ankle joint. Hence, the robotic orthosis can assist a patient in a natural gait close to a normal gait, can assist a patient in daily life, and can maximize rehabilitation effects.

In addition, the robotic orthosis for a lower extremity for gait rehabilitation training according to the embodiments of the present invention is a soft wearable device made of a soft material, thus being lightweight, flexible, well wearable, and safe.

In addition, the robotic orthosis for a lower extremity for gait rehabilitation training according to the embodiments of the present invention can recognize a location of a patient and is movable by being supplied with compressed air through a pneumatically powered luggage carrier capable of autonomous driving. Hence, the compressed air can be supplied depending on a patient's gait cycle during the patient's walking.

In addition, the robotic orthosis for a lower extremity for gait rehabilitation training according to the embodiments of the present invention can obtain the patient's gait condition and gait pattern in real time by using an inertial measurement module and a wireless plantar pressure insole module and can use the gait condition and the gait pattern for real-time control to operate a pneumatic artificial muscle at a special gait point and phase.

Further, the robotic orthosis for a lower extremity for gait rehabilitation training according to the embodiments of the present invention can be applied to customized rehabilitation through feedback of quantitative gait data and gait indexes by acquiring and analyzing a patient's gait data.

### Brief Description of Drawings

FIG. 1 is a perspective view illustrating an example of a robotic orthosis for a lower extremity for gait rehabilitation training put on a user according to an embodiment of the present invention.
FIG. 2 is a plan view illustrating a structure of a knee stretching member according to the embodiment of the present invention.
FIG. 3A is a conceptual view illustrating an operation of the knee stretching member in an initial and mid swing phase of the knee stretching member according to the embodiment of the present invention.
FIG. 3B is a conceptual view illustrating an operation of the knee stretching member in a terminal swing phase of the knee stretching member according to the embodiment of the present invention.
FIG. 3C is a conceptual view illustrating an operation of the knee stretching member in a stance phase of the knee stretching member according to the embodiment of the present invention.
FIG. 4 is a plan view illustrating a first ankle supporting member according to the embodiment of the present invention.
FIG. 5Ais a conceptual view illustrating a state in which air is yet to be injected into the first ankle supporting member according to the embodiment of the present invention.
FIG. 5B is a conceptual view illustrating a state in which air is injected into the first ankle supporting member according to the embodiment of the present invention.
FIG. 5C is a conceptual view illustrating an example in which the first ankle supporting member supports an ankle at an inversion side according to the embodiment of the present invention.
FIG. 5D is a conceptual view illustrating an example in which the first ankle supporting member supports the ankle at an eversion side according to the embodiment of the present invention.
FIG. 6 is a plan view illustrating an artificial muscle pack according to the embodiment of the present invention.
FIG. 7A is a conceptual view illustrating an example of a state in which air is yet to be injected into air chambers at both sides of a shin and a calf according to the embodiment of the present invention.
FIG. 7B is a conceptual view illustrating an example of a state in which air is injected into the air chamber at the side of the shin according to the embodiment of the present invention.
FIG. 7C is a conceptual view illustrating an example of a state in which air is injected into the air chamber at the side of the calf according to the embodiment of the present invention.
FIG. 8 is a perspective view illustrating a luggage carrier according to the embodiment of the present invention.
FIG. 9 is a block diagram illustrating a configuration in the luggage carrier according to the embodiment of the present invention.
FIG. 10 is a conceptual view illustrating an example in which an inertial measurement module and an insole module are installed according to the embodiment of the present invention.
FIG. 11 is a conceptual diagram illustrating angles between ground and a thigh, a shin, and a dorsum of a foot, angles of a hip, a knee, and an ankle joint, and locations of a knee, an ankle, and a tiptoe according to the embodiment of the present invention.
FIG. 12 is a table illustrating generation of an algorithm and a control force in each gait phase according to the embodiment of the present invention.
FIG. 13 illustrates photographs displaying a wearing order of the robotic orthosis for a lower extremity for gait rehabilitation training according to an embodiment of the present invention.

### [Description of Reference Numbers]

100: Robotic orthosis for lower extremity for gait rehabilitation training
10: Knee stretching member
13: Knee sleeve
15: Knee supporting chamber
18: Velcro
20: First ankle supporting member
21: Ankle sleeve
25: Ankle supporting chamber
28: Velcro
30: Second ankle supporting member
31: Shin sleeve
32: Guard
34: Artificial muscle pack
40: Luggage Carrier
41: Handle
41a: Camera
41b: Laser distance sensor
42: Air compressor
43: Air tank
44: Pressure regulator
45: Battery
46: Loadcell amplifier
47: IMU receiver
48: Main controller
49: Self-driving unit
50: Inertial measurement module
60: Insole module
70: Air hose
71: Waist belt

### Mode for Invention

Hereinafter, embodiments disclosed in this specification will be described in detail with reference to the accompanying drawings; however, the same or similar reference signs are assigned to the same or similar configurational elements, and the repeated description thereof is to be omitted.

In the description of the embodiments disclosed in this specification, when the specific description of a known technology related to the embodiments is considered to obscure the gist of the embodiments disclosed in this specification, the detailed description thereof is to be omitted. In addition, the accompanying drawings are only provided to enable the embodiments disclosed in this specification to be easily understood, and thus the technical idea disclosed in this specification is not limited to the accompanying drawings. All modifications, equivalents, and alternatives included in the technical idea and technical scope of the present invention are to be construed to be part of the present invention.

Terms having an ordinal number such as first or second can be used in describing various configurational elements; however, the configurational elements are not limited to the terms. The terms are used only for a purpose of distinguishing one configurational element from another configurational element.

The description in which one configurational element is mentioned to be "connected to" another configurational element is to be understood to mean that the one configurational element can be directly connected to the other configurational element or that still another configurational element can be present therebetween.

Singular expression also includes plural expression thereof, unless obviously implied otherwise in context.

In this specification, a term such as "to comprise" or "to have" is construed to specify that a feature, a number, a step, an operation, a configurational element, a part, or an assembly thereof described in the specification is present and not to exclude a possibility of presence or addition of one or more other features, numbers, steps, operations, configurational elements, parts, or assemblies thereof in advance.

In this specification, a term such as "unit", "module", or "controller" which includes a control process can indicate not only hardware but also an assembly of hardware and software driven by the hardware. For example, the hardware can be a data processing unit including a CPU or another processor. In addition, the software driven by the hardware can be a program such as a running process, an object, an executable file, a thread of execution, or a computational program.

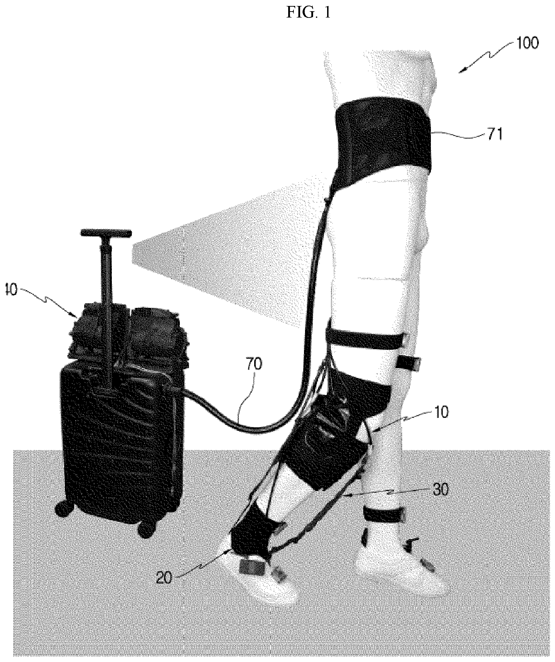

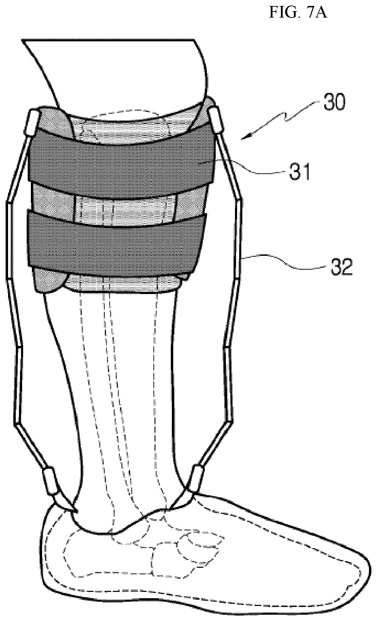

With reference to FIGS. 1 to 7C, a robotic orthosis 100 for a lower extremity for gait rehabilitation training according to the embodiments of the present invention is described.

The robotic orthosis 100 for a lower extremity for gait rehabilitation training according to the embodiments includes a knee stretching member 10.

The knee stretching member 10 is provided to be installable on a knee to maintain an extension state, that is, a stretched state, of the knee.

The knee stretching member 10 enables a knee joint to be stretched in a swing phase and enables a state in which the knee is stretched to be maintained in a stance phase, thus enabling an affected leg to support body weight.

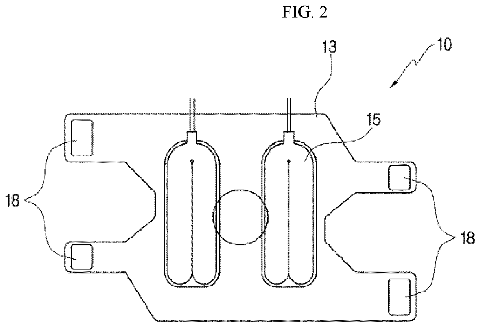

As illustrated in FIG. 2, the knee stretching member 10 includes a knee sleeve 13 and a knee supporting chamber 15.

The knee sleeve 13 is provided to surround the knee joint. Desirably, the knee sleeve 13 is made of a soft material such as an elastic material.

The knee sleeve 13 can have patches of Velcro 18 at both ends thereof, and the patches of Velcro 18 enable the knee sleeve 13 to surround and fix a leg around a patient's knee joint. FIG. 2 illustrates an example in which the two patches of Velcro 18 are attached to each of right and left ends of the knee sleeve 13.

Positions of the patches of Velcro 18 or positions at which the patches of Velcro 18 are attached to each other can be adjusted depending on a body size.

The knee supporting chamber 15 is mounted to be connected to the knee sleeve 13 and supports the knee joint such that the knee is stretched when air is injected therein and is inflated.

The knee supporting chamber 15 can be manufactured of a soft material such as a soft cloth material and, desirably, can be manufactured of a cloth material coated with polyurethane or the like.

When air is injected into the knee supporting chamber 15, the knee supporting chamber 15 is inflated so as to support the knee joint. In addition, when the air injected into the knee supporting chamber 15 is discharged, the knee supporting chamber is deformed into a flat shape and does not affect movement of the knee joint.

In a state in which air is injected into the knee supporting chamber 15 and is inflated, the knee supporting chamber 15 is inflated and is tight while being deformed into a bar shape.

When the knee supporting chamber 15 is inflated in the terminal swing phase, the knee supporting chamber 15 enables the knee joint to be extended in a direction parallel to the leg.

In addition, in the stance phase, the knee supporting chamber 15 maintains pressure therein to enable the state in which the leg is straightly stretched to be maintained such that the affected leg can support the weight. In addition, the knee supporting chamber 15 assists rigidity of the knee j oint, thereby enabling improvement of gait stability and injury prevention in the stance phase.

A degree of assisting strength of the knee stretching member 10 can be controlled by adjusting flexural rigidity of the knee supporting chamber 15 based on the pressure intensity of air injected into the knee supporting chamber 15.

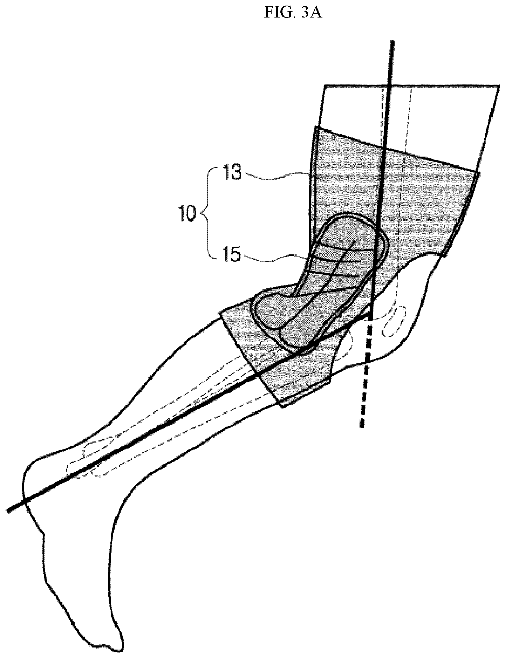

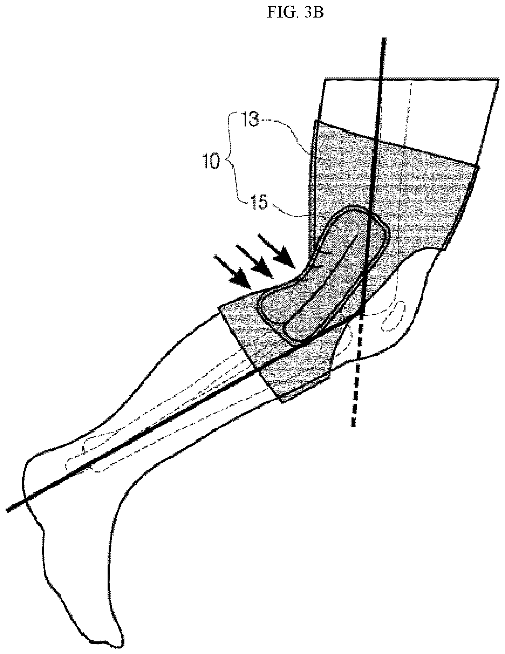

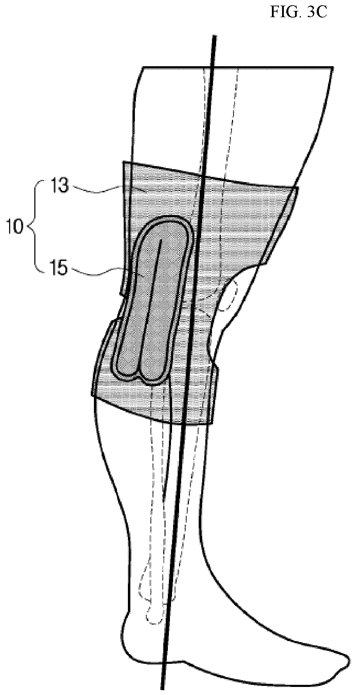

FIGS. 3A to 3C illustrate an example in which air is not injected into the knee supporting chamber 15 in the initial and mid swing phase and the knee supporting chamber 15 is inflated in the direction parallel to the leg to assist stance in the terminal swing phase and enables the state in which the leg is straightly stretched to be maintained in the stance phase.

In the embodiments of the present invention, as described above, both the knee sleeve 13 and the knee supporting chamber 15 are made of the soft material, so extension and flexion of the knee according to a gait cycle can be repeated without restriction.

In some of the embodiments, the robotic orthosis 100 for a lower extremity for gait rehabilitation training of the present invention can further include at least one of a first ankle supporting member 20 and a second ankle supporting member 30.

In the embodiments, the first ankle supporting member 20 is installed at an ankle so as to inhibit inversion or eversion bending of the ankle.

The first ankle supporting member 20 can include an ankle sleeve 21 and an ankle supporting chamber 25.

The ankle sleeve 21 is provided to surround an ankle joint. Desirably, the ankle sleeve 21 is made of an elastic material.

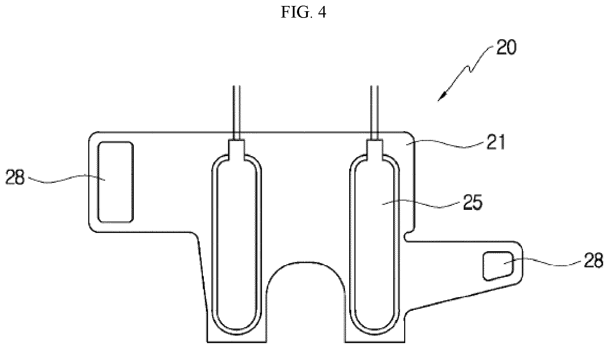

The ankle sleeve 21 can have patches of Velcro 28 at both ends thereof, and the patches of Velcro 28 enable the ankle sleeve 21 to surround and fix the patient's ankle joint. FIG. 4 illustrates an example in which one patch of Velcro 28 is attached to each of right and left ends of the ankle sleeve 21.

Positions of the patches of Velcro 28 on the ankle sleeve 21 or positions at which the patches of Velcro 28 are attached to each other can be adjusted depending on a body size.

The ankle supporting chamber 25 can be mounted to be connected to the ankle sleeve 21 and can support the ankle joint when air is injected therein and is inflated.

The inflated ankle supporting chamber 25 has an increase in rigidity so as to inhibit excessive inversion or eversion bending of the ankle.

Before the affected leg, on which the first ankle supporting member 20 is installed, reaches the ground, air is injected into the ankle supporting chamber 25 and to inflate the ankle supporting chamber in advance, and thereby rigidity of the ankle in inversion and eversion directions can be reinforced. In this manner, the impact applied to the ankle in the initial stance phase in which the affected leg comes into contact with the ground is absorbed, and gait stability is improved.

In addition, the first ankle supporting member 20 enables the pressure of the ankle supporting chamber 25 to be maintained in the stance phase so as to maintain a state in which the ankle is straightly stretched such that the affected leg can support the weight and a sprain and a falling accident due to bending of the ankle can be prevented.

Intensity of an ankle supporting force of the first ankle supporting member 20 which supports the ankle can be controlled by adjusting flexural rigidity of ankle support based on pressure intensity of air injected into the ankle supporting chamber 25.

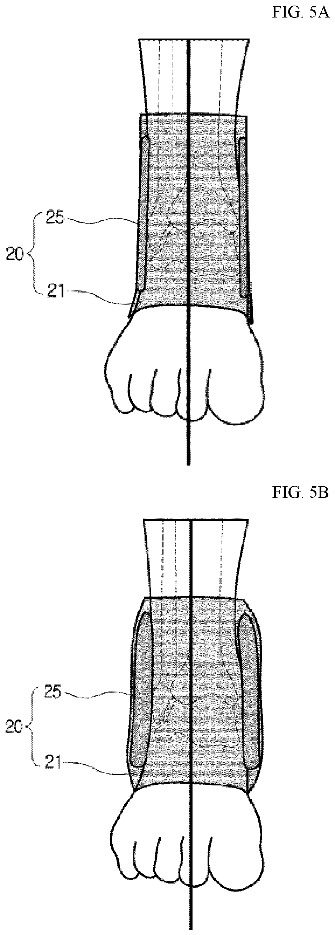

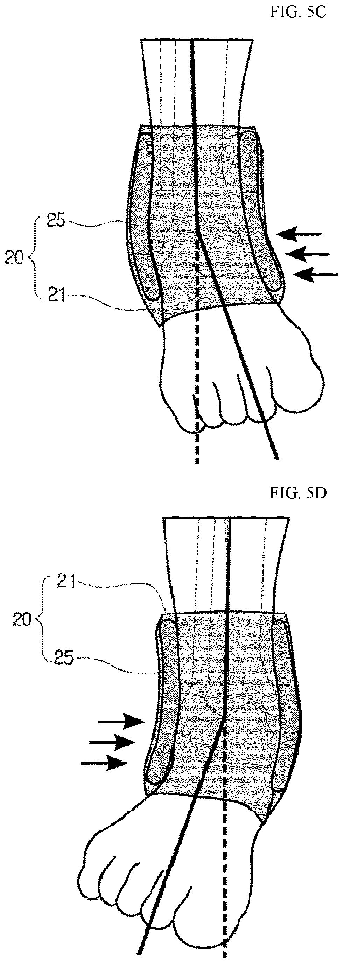

FIGS. 5C and 5D illustrate an example in which the first ankle supporting member 20 supports the ankle at inversion and eversion sides.

The ankle supporting chamber 25 can be manufactured of a cloth material and, desirably, can be manufactured of a cloth material coated with polyurethane.

In the embodiment, the second ankle supporting member 30 can include a shin sleeve 31, guards 32, and artificial muscle packs 34.

The shin sleeve 31 can be provided to surround a shin.

The shin sleeve 31 can have patches of Velcro at both ends thereof, and the patches of Velcro enable the ankle sleeve 21 to surround and fix the vicinity of the patient's shin.

The guards 32 can be installed at both the shin and a calf, respectively.

The guards 32 can be attached to fix an upper part of the artificial muscle packs 34 on the shin sleeve 31. Desirably, the guard 32 is made of a hard material. The guard 32 can have a Velcro tie such that the Velcro tie can be tied on the shin and the calf, and thereby the guard 32 can be fixed.

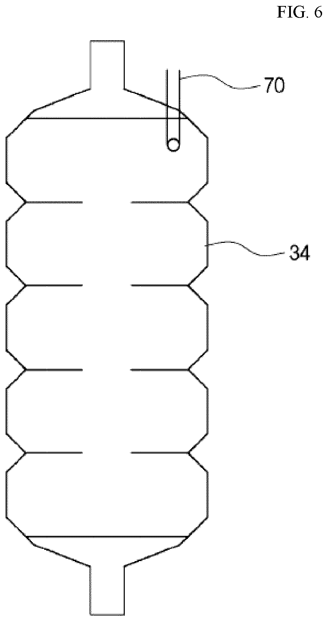

The artificial muscle packs 34 can be connected to the guards 32 and can have a plurality of air chambers therein as illustrated in FIG. 6.

The air chambers can be installed at both sides of the shin and the calf.

A plurality of air chambers can be provided and can be connected in series in a direction parallel to the leg. The air chamber maintains a flat shape when air is discharged. When compressed air is injected into the air chamber, the air chamber is inflated and is contracted in a length direction thereof so as to generate a protective force at the calf and the shin.

The artificial muscle pack 34 can be manufactured of a cloth material coated with polyurethane.

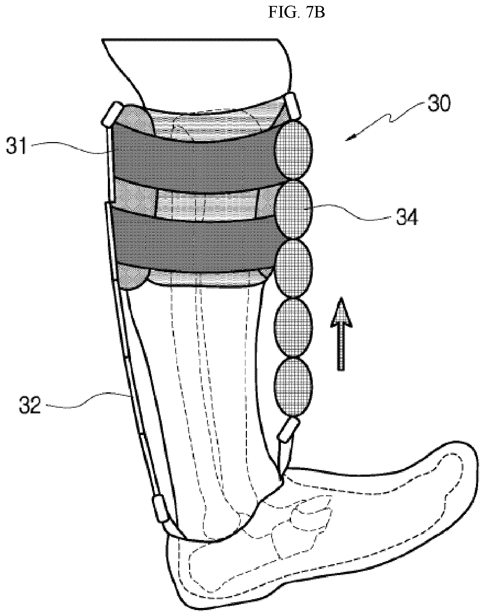

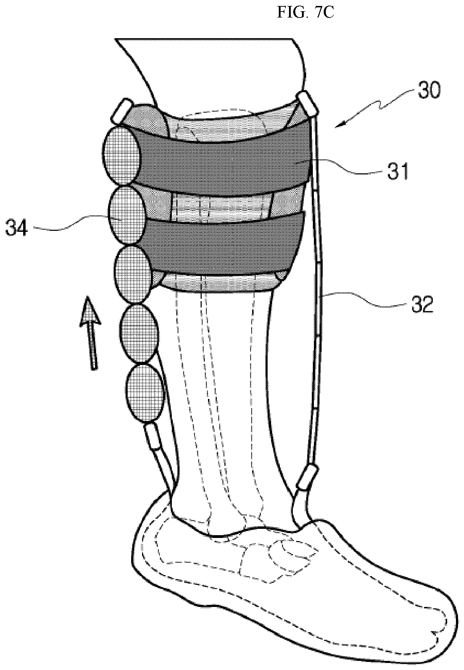

The artificial muscle packs 34 can have the air chambers at the sides of the shin and the calf. The artificial muscle packs 34, with reference to FIGS. 7A and 7B, cause the air chamber installed at the side of the shin to assist a motion of dorsiflexion of a foot and, with reference to FIG. 7C, cause the air chamber installed at the side of the calf to assist plantarflexion.

The artificial muscle packs 34 assist the motion of the dorsiflexion of the ankle joint so as to inhibit foot drop and enable sufficient foot clearance to be secured by injecting the compressed air into the air chamber installed at the side of the shin in the swing phase and inflating the air chambers.

In addition, the artificial muscle packs 34 assist the motion of the dorsiflexion even during initial contact, which is a time when the foot starts to reach the ground, so as to absorb and soften the impact occurring when the foot reaches the ground.

On the other hand, air in the air chamber attached to the shin is emitted in the stance phase, and the compressed air is injected into the air chamber attached to the calf on a rear surface of the leg such that the air chamber is inflated to assist the motion of the plantarflexion of the ankle joint. Hence, while the foot kicks the ground, thrust is generated.

Intensity of a support force of the second ankle supporting member 30 which supports the shin and the calf can be controlled by adjusting flexural rigidity of shin and calf support based on pressure intensity of air injected into air chambers.

As an example, in a state in which a sole is in contact with the ground, air in the air chamber installed at the side of the shin can be discharged outside, and air can be injected into the air chamber installed at the side of the calf.

In addition, in a state in which a sole is separated from the ground, air can be injected into the air chamber installed at the side of the shin, and air in the air chamber installed at the side of the calf can be discharged outside.

The second ankle supporting member 30 can further include a Velcro tie and a buckle.

The Velcro tie can surround and fix the guards 32.

The buckle is installed at a foot accommodating portion and combines the artificial muscle packs 34.

In some of the embodiments, the robotic orthosis 100 for a lower extremity for gait rehabilitation training of the present invention can further include a luggage carrier 40. The luggage carrier can be an autonomous driving luggage carrier, particularly, and thereby the compressed air can be supplied while the luggage carrier moves together with the patient's walking. Hereinafter, the specific description thereof will be provided.

The luggage carrier 40 can generate compressed air and can supply the generated compressed air to at least one of the knee stretching member 10 and the first and second ankle supporting members 30. In this respect, the luggage carrier can further include a compressor that generates compressed air and one or more hoses for supplying the generated compressed air to at least one of the knee stretching member 10 and the first and second ankle supporting members 30.

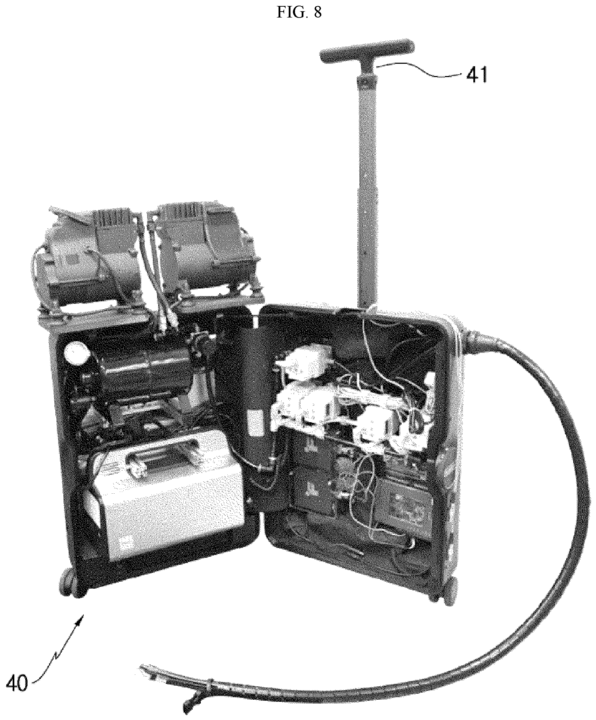

To be more specific with reference to FIG. 8, the luggage carrier 40 can have a handle 41, and a camera 41a and a laser distance sensor 41b are installed on the handle 41 so as to enable the luggage carrier 40 to recognize a location of a patient and move by a self-driving unit 49 to follow the patient. Hence, the luggage carrier 40 according to the embodiment of the present invention can perform autonomous driving.

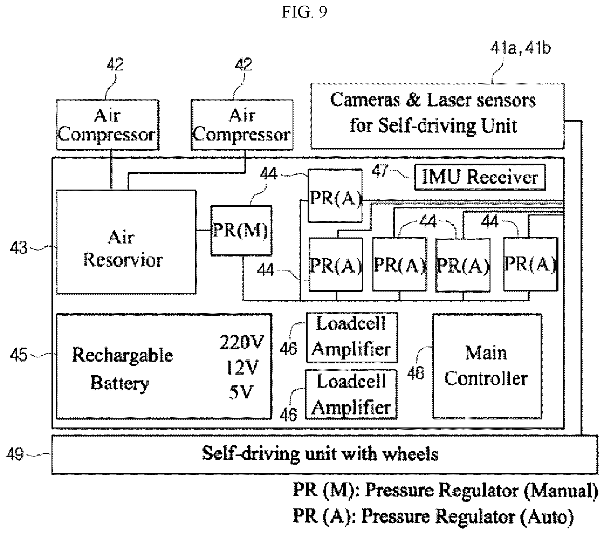

In addition, FIG. 9 is a block diagram illustrating a configuration in the luggage carrier 40. With reference to FIG. 9, the luggage carrier 40 contains an air compressor 42 that generates compressed air, an air tank 43 that stores the compressed air, a pressure regulator 44 that regulates intensity of air pressure, a battery 45 for electric power supply, a loadcell amplifier 46 for collecting a sensor signal, an IMU receiver 47, and a main controller 48.

The main controller 48 analyzes in real time the gait cycle and pattern from a signal measured in an inertial measurement module 50 to be described below and controls the pressure regulator 44 to supply the compressed air having set pressure to a driver at an appropriate time.

In addition, the main controller 48 monitors a gait condition by communicating with an external desktop or laptop and enables external control to be performed.

In the embodiment, the robotic orthosis 100 for a lower extremity for gait rehabilitation training according to the embodiment of the present invention can further include the inertial measurement module 50.

The inertial measurement module 50 checks the patient's gait condition and gait pattern in real time based on an angle between the ground and at least one of the thigh, the shin, and the dorsum of the foot during the patient's walking so as to enable the main controller 48 to control an operation of at least one of the knee stretching member 10 and the first and second ankle supporting members 30.

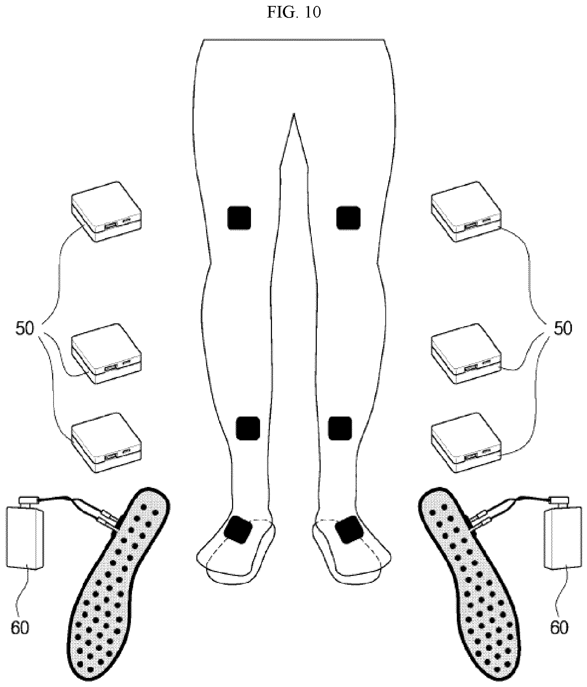

In this respect, with reference to FIG. 10, the inertial measurement module 50 can be installed on at least one of the patient's thigh, shin, and dorsum of the foot.

For example, the inertial measurement module 50 can be an inertial measurement unit (IMU).

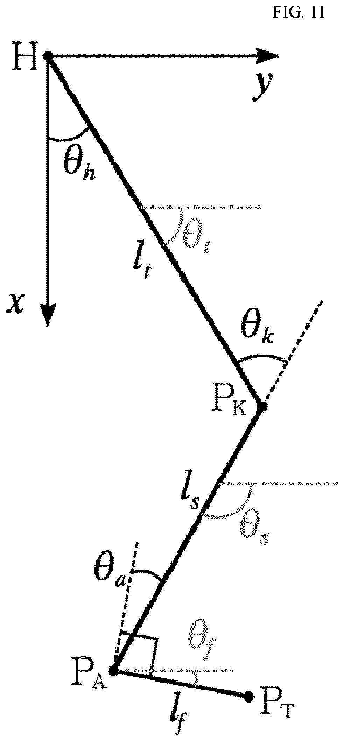

For example, with reference to FIG. 11, the inertial measurement module 50 can measure an angle θₜ, θₛ, or θ_{f} between the ground and at least one of the thigh, the shin, and the dorsum of the foot, and then can calculate an angle θₕ, θₖ, or θₐ of a hip, a knee, or an ankle joint by using a kinematics model of a lower extremity. In addition, with a location H of a hip joint as an origin point, locations P_{K}, P_{A}, and P_{T} of the knee, the ankle, and a tiptoe can be calculated.

On the other hand, the luggage carrier 40 can include a wireless plantar pressure insole module 60 so as to measure whether both soles are in contact with the ground in real time.

In addition, the inertial measurement module 50 enables the main controller 48 to determine a gait cycle as any one of a loading response phase, a mid stance phase, a terminal stance phase, a pre-swing phase, an initial swing phase, a mid swing phase, and a terminal swing phase based on a posture of the patient's both lower extremities and whether the patient's soles are in contact with the ground.

As described above, the luggage carrier 40 can supply the compressed air to the knee stretching member 10 in the terminal swing phase, the loading response phase, the mid stance phase, and the terminal stance phase and supply the compressed air to the first ankle supporting member 20 in the terminal swing phase, the loading response phase, and the terminal stance phase.

As described above, the patient's gait condition and gait pattern can be checked in real time by using sensing information measured and calculated in real time, and the patient's gait condition and gait pattern can be used in real-time control to operate pneumatic artificial muscle at a specific gait point and phase. Further, the patient's gait data can be acquired and analyzed so as to feedback quantitative gait data and gait indexes and thereby can be used in customized rehabilitation.

In addition, the robotic orthosis 100 for a lower extremity for gait rehabilitation training can further include an air hose 70 that enables the compressed air generated in the luggage carrier 40 to be transmitted to the knee stretching member 10, the first ankle supporting member 20, and second ankle supporting member 30.

One end side of the air hose 70 communicates with the air tank 43, and the other end side thereof communicates with each of the knee supporting chamber 15, the ankle supporting chamber 25, and the artificial muscle packs 34 so as to supply compressed air in the air tank 43 to each of the knee stretching member 10, the first ankle supporting member 20, and the second ankle supporting member 30.

The air hose 70 can include a waist belt 71 for branching into the knee supporting chamber 15, the ankle supporting chamber 25, and the artificial muscle packs 34, and the air hose 70 can be fixed to the patient's waist with the waist belt 71.

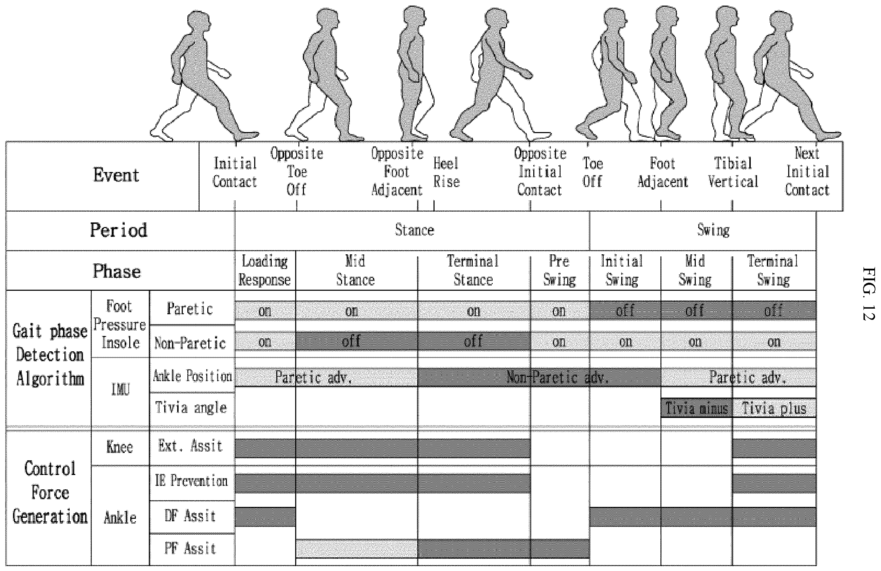

In the embodiment of the present invention, the gait phase will be described hereinafter. Regarding the gait phase, FIG. 12 illustrates generation of an algorithm and a control force in each gait phase.

The gait phase largely includes the stance phase in which a foot is in contact with the ground and the swing phase in which the foot is separated from the ground, and the gait phase includes seven phases in total depending on whether relative locations of both legs are in contact with the ground.

The seven phases of the stance phase and the swing phase are divided into a loading response phase, a mid stance phase, a terminal stance phase, a pre-swing phase, an initial swing phase, a mid swing phase, and a terminal swing phase.

A phase to which a gait condition belongs during walking can be determined in real time by detecting posture of both lower extremities and whether the soles are in contact with the ground by using the inertial measurement module 50 included in the embodiment of the present invention, and determination criteria are as follows.

The loading response phase corresponds to a case where both feet are in contact with the ground, and an affected ankle joint is located in front of an unaffected ankle joint.

The mid stance phase corresponds to a case where an affected foot is in contact with the ground, an unaffected foot is separated from the ground, and the affected ankle joint is located in front of the unaffected ankle joint.

The terminal stance phase corresponds to a case where the affected foot is in contact with the ground, the unaffected foot is separated from the ground, and the affected ankle joint is located behind the unaffected ankle joint.

The pre-swing phase corresponds to a case where both feet are in contact with the ground, and the affected ankle joint is located behind the unaffected ankle joint.

The initial swing phase corresponds to a case where the affected foot is separated from the ground, the unaffected foot is in contact with the ground, and the affected ankle joint is located behind the unaffected ankle joint.

The mid swing phase corresponds to a case where the affected foot is separated from the ground, the unaffected foot is in contact with the ground, and the affected ankle joint is located in front of the unaffected ankle joint. In addition, the mid swing phase corresponds to a case where the knee joint is located in front of the ankle.

The terminal swing phase corresponds to a case where the affected foot is separated from the ground, the unaffected foot is in contact with the ground, and the affected ankle joint is located in front of the unaffected ankle joint. In addition, the terminal swing phase corresponds to a case where the knee joint is located behind the ankle.

Hereinafter, regarding the configurations of the knee stretching member 10, the first ankle supporting member 20, and the second ankle supporting member 30, a control method for generating a control force will be described.

The knee stretching member 10 enables the knee joint to be stretched in the terminal swing phase. In addition, the knee stretching member 10 enables a state in which the knee is stretched in the terminal stance phase to be maintained in the loading response phase, thus enabling the affected leg to support body weight.

The first ankle supporting member 20 reinforces the rigidity of the ankle joint in the terminal swing phase in advance against the impact generated when the foot reaches the ground. In addition, in the loading response phase, the first ankle supporting member inhibits excessive inversion and eversion bending of the ankle joint in the terminal stance phase and reinforces the rigidity of the ankle joint in the inversion and eversion directions, thereby absorbing the impact applied to the ankle and improving the gait stability.

The second ankle supporting member 30 assists the motion of the dorsiflexion of the ankle joint in the swing phase so as to inhibit foot drop and enables sufficient foot clearance to be secured.

In addition, the second ankle supporting member 30 assists the motion of the dorsiflexion in the loading response phase so as to absorb the impact applied to the ankle, thus improving the gait stability.

In addition, the second ankle supporting member 30 assists the plantarflexion from the mid swing phase to the pre-swing phase such that thrust is generated while the foot kicks the ground.

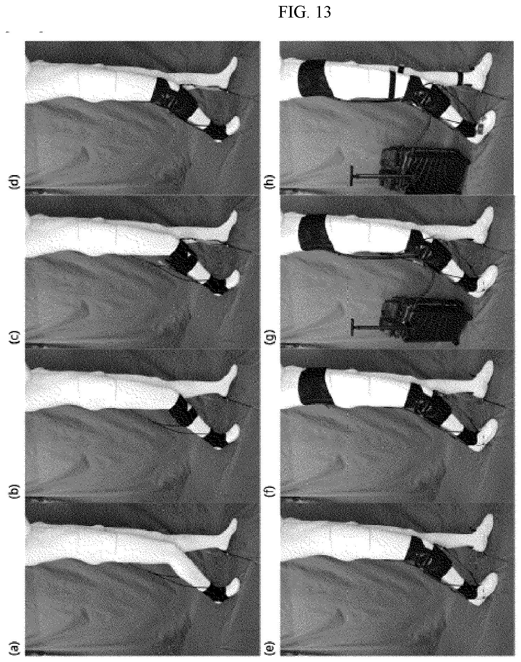

Hereinafter, with reference to a to h in FIG. 13, a wearing order of the robotic orthosis 100 for a lower extremity for gait rehabilitation training according to the embodiment of the present invention will be described.

Insoles for fixing a plantar pressure sensor and the artificial muscle packs 34 are inserted into indoor shoes. Then, a sensor module is powered up.

With reference to a in FIG. 13, the first ankle supporting member 20 is put on the affected ankle. In addition, the indoor shoes are put on both feet.

Then, with reference to b and c in FIG. 13, the shin sleeve 31 of the second ankle supporting member 30 is put on, the guards 32, to which the artificial muscle packs 34 of the second ankle supporting member 30 are connected, are put on the shin and the calf, and fixing is performed with a Velcro tie.

With reference to d in FIG. 13, the knee stretching member 10 is put on.

With reference to e in FIG. 13, terminal edges of the artificial muscle packs 34 of the second ankle supporting member 30 are connected to a buckle attached to the insole.

With reference to f in FIG. 13, a waist belt, to which the air hose is connected and is branched, is put on.

With reference to g in FIG. 13, the luggage carrier 40 that can perform the autonomous driving and the waist belt are connected to each other with a bundle of air hoses, and air hoses are branched from the bundle of air horses attached to the waist belt so as to be connected to the driving units, respectively.

With reference to h in FIG. 13, the wireless inertial measurement module 50 is attached to at least one of both thighs, shins, and dorsum parts of the feet, and wireless modules for plantar pressure measurement are attached to indoor shoes.

The robotic orthosis 100 for a lower extremity for gait rehabilitation training described above is not limited to the configurations and methods of the embodiments described above but can have a configuration in which all or a part of individual embodiments are selectively combined such that the embodiment can be variously modified.

It is obvious for those skilled in the art to realize still another specific example within a range not departing from the idea and the essential feature of the present invention. Consequently, the detailed description is not to be construed to be limited in any aspect but is considered an exemplary example. The scope of the present invention is determined through reasonable interpretation of the accompanying claims, and any modifications within an equivalent range of the present invention are included in the scope of the present invention.

### Industrial Applicability

According to the embodiments of the present invention, a robotic orthosis for a lower extremity for gait rehabilitation training can assist a patient in a natural gait close to a normal gait by enabling a knee joint of a paraplegic patient such as a post-stroke hemiplegic patient to be extended, flexed, or the like according to a gait cycle of the patient when a patient walks around. Hence, the robotic orthosis can assist a paraplegic patient in daily life and can maximize rehabilitation effects.

## Claims

1. A robotic orthosis for a lower extremity for gait rehabilitation training, comprising:
a knee stretching member which is provided to be installable on a knee so as to enable a knee joint to be stretched in a swing phase and enable a state in which the knee is stretched to be maintained in a stance phase,
wherein the knee stretching member comprises:
a knee sleeve surrounding the knee joint; and
a knee supporting chamber which is mounted so as to be connected to the knee sleeve, and which, when air is introduced therein and is inflated in the swing phase, enables the knee to be stretched by supporting the knee joint, and enables the state in which the knee is stretched to be maintained in the stance phase.

2. The robotic orthosis for a lower extremity for gait rehabilitation training according to claim 1, further comprising:
a luggage carrier that generates compressed air and supplies the generated compressed air to the knee stretching member.

3. The robotic orthosis for a lower extremity for gait rehabilitation training according to claim 2,
wherein the luggage carrier is an autonomous driving luggage carrier.

4. The robotic orthosis for a lower extremity for gait rehabilitation training according to claim 1,
wherein the knee sleeve and the knee supporting chamber are made of a soft material.

5. The robotic orthosis for a lower extremity for gait rehabilitation training according to claim 1, further comprising at least one of:
a first ankle supporting member that is installed at an ankle so as to inhibit inversion or eversion bending of the ankle, and
a second ankle supporting member that is installed on at least one of a shin and a calf such that an ankle is flexed toward a dorsum or a sole of a foot.

6. The robotic orthosis for a lower extremity for gait rehabilitation training according to claim 5,
wherein the first ankle supporting member comprises:
an ankle sleeve surrounding an ankle j oint; and
an ankle supporting chamber which is mounted so as to be connected to the ankle sleeve, and which, when air is introduced therein and is inflated, supports the ankle joint.

7. The robotic orthosis for a lower extremity for gait rehabilitation training according to claim 5,
wherein the second ankle supporting member comprises:
a shin sleeve surrounding a shin;
guards that are installed at both the shin and the calf, respectively; and
artificial muscle packs that are connected to the respective guards and have a plurality of air chambers inside.

8. The robotic orthosis for a lower extremity for gait rehabilitation training according to claim 7,
wherein the second ankle supporting member further comprises:
a Velcro tie that surrounds and secures the guards; and
a buckle that is installed at a foot accommodating portion and combines the artificial muscle packs.

9. The robotic orthosis for a lower extremity for gait rehabilitation training according to claim 7,
wherein the air chambers are installed at both sides of the shin and the calf, respectively,
wherein, in a state in which a sole is in contact with ground, air in the air chamber installed at the side of the shin is discharged outside, and air is injected into the air chamber installed at the side of the calf, and
wherein, in a state in which a sole is separated from the ground, air is injected into the air chamber installed at the side of the shin, and air in the air chamber installed at the side of the calf is discharged outside.

10. The robotic orthosis for a lower extremity for gait rehabilitation training according to claim 5, further comprising:
a luggage carrier that generates compressed air and is capable of supplying the generated compressed air to at least one of the knee stretching member and the first and second ankle supporting members.

11. The robotic orthosis for a lower extremity for gait rehabilitation training according to claim 10, further comprising:
an inertial measurement module that is installed on at least one of a patient's thigh, shin, and dorsum of a foot and checks the patient's gait condition and gait pattern in real time based on an angle between ground and at least one of the thigh, the shin, and the dorsum of the foot during the patient's walking so as to control an operation of at least one of the knee stretching member and the first and second ankle supporting members.

12. The robotic orthosis for a lower extremity for gait rehabilitation training according to claim 11,
wherein the inertial measurement module is capable of determining a gait phase as any one of a loading response phase, a mid stance phase, a terminal stance phase, a pre-swing phase, an initial swing phase, a mid swing phase, and a terminal swing phase based on a posture of the patient's both lower extremities and whether the patient's soles is in contact with the ground.

13. The robotic orthosis for a lower extremity for gait rehabilitation training according to claim 12,
wherein the luggage carrier supplies
the compressed air to the knee stretching member in the terminal swing phase, the loading response phase, the mid stance phase, and the terminal stance phase, and
the compressed air to the first ankle supporting member in the terminal swing phase, the loading response phase, and the terminal stance phase.
